# EUROPEAN PATENT APPLICATION

(11) **EP 1 902 736 A1**
(43) Date of publication of application: **26.03.2008**
(21) Application number: 06076744.9
(22) Date of filing: 19.09.2006
(51) Int. Cl.: A61L 9/12

(54) **Dual scent air-freshening dispensing device**

(71) Applicant: Sara Lee/DE N.V., 3532 AA Utrecht (NL)
(72) Inventor: Bulsink, Dirk Jan, 2331 BZ Leiden (NL); Bogerd, Caspar Barry, 2761 GA Zevenhuizen (NL)
(74) Representative: van Loon, C.J.J.

(57) **Abstract**

The invention relates to an air-freshening dispensing device, comprising: a housing, comprising a pair of mounts for mounting respective containers provided in outer sections of said housing; and a switchable shutter element arranged for selectively shutting access of an airflow to any of the outer sections. According to the invention, said shutter element is provided in a mid section between said outer sections and communicatively coupled to an air inlet. An air outlet provides throughput of the airflow through the mid section and the device further comprises a clamp for clamping the unit onto an outlet channel to provide an airflow into the air inlet.

## Description

The invention relates to an air-freshening dispensing device. In particular, it relates to a dispensing device for delivering a fragrance, for example a deodorising agent or a fragrance, for instance in an automobile.

W02003/028775 discloses a dispensing unit for dispensing a combination of fragrances, wherein a pair of mounts is provided for mounting respective fragrance containers in outer sections of the device. A switchable shutter element is arranged for selectively shutting access of the airflow to any of the outer sections. The device is dependent on a reversal of air stream flow of a fan to provide any of a selected fragrance. This mechanism is not practical for applications which are used in conjunction with an external fan, for instance, in automobiles.

EP200476393 discloses an air-freshening dispensing device for a vehicle. The device can be clamped onto an outlet channel of a car fan and comprises a mount for mounting a fragrance container. The device only comprises a single fragrance container.

It is desirable to provide a duo fragrance dispensing device which is compact and provides a possibility to selectable fragrance.

In accordance with an aspect of the present invention there is provided an air-freshening dispensing device according to the features of claim 1. In particular, there is provided an air-freshening dispensing device, comprising: a housing, comprising a pair of mounts for mounting respective containers provided in outer sections of said housing; and a switchable shutter element arranged for selectively shutting access of an airflow to either of the outer sections; wherein said shutter element is provided in a mid section between said outer sections; said mid section being communicatively coupled to an air inlet, and further comprising an air outlet for providing throughput of the airflow through the mid section; the device further comprising a clamp for clamping the unit onto an air outlet channel to provide an airflow into the air inlet.

A device thus configured may provide compact and selective switching, wherein, when in use, only one fragrance is selected and the other is shut off from the air flow. Accordingly, a cost effective and efficient device for providing multiple fragrances can be provided.

Further features and benefits can be derived from the description, read in conjunction with the figures. In the figures:
Figure 1 shows a schematic perspective view that is partly shown in cross sectional view, of a dispenser according to an aspect of the invention;
Figures 2A and B show alternative working conditions wherein a shutter element alternatively provides and shuts access to an evaporation surface of a fragrance;
Figure 3 shows a schematic perspective cross sectional view of the dispensing device of Figure 1 sectioned along line I-I, showing the shutter in a selected shutting position; and
Figure 4 shows a top view of the slider through a top cross sectional view of the dispenser of Figure 1 sectioned along line II-II.

In the figures, the same or corresponding items are referenced by the same reference numerals.

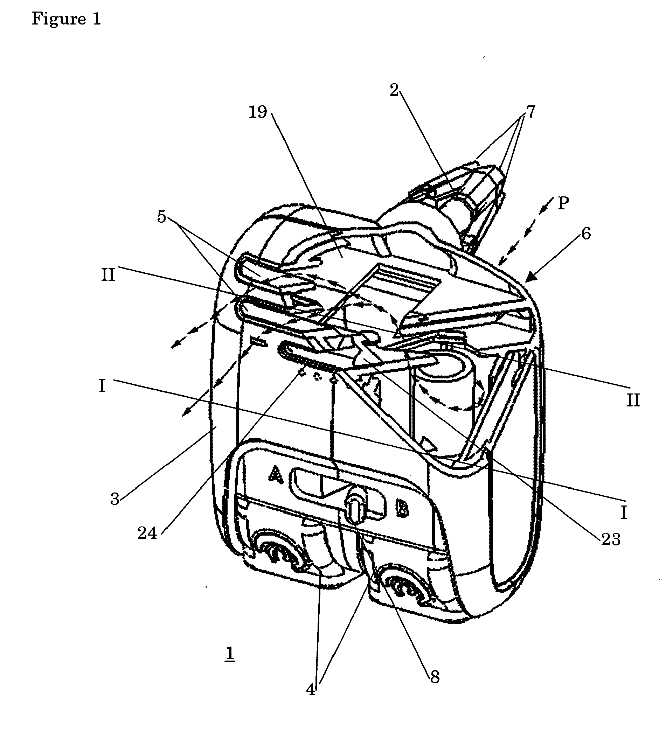

In Figure 1 a front perspective schematic view of an embodiment of the dispensing device or air freshener 1 according to the invention is illustrated. The air-freshener 1 is attachable to a site in the interior of a vehicle, in particular a motorcar, in a conventional way, for example, by the clamping mechanism 2 explained below. The air-freshener 1 is provided with a housing 3 into which containers 4 for a volatile liquid can be inserted (indicated by references A and B in the figure). The front comprises ventilation outlets 5 via which air can flow through the air freshener 1.

The rear comprises ventilation openings 6, that allow air to be transported into the housing 3. A clamping element 2 is present for securing the air-freshener 1 on a strip-shaped element, in particular that of a guard of the fan in the dashboard of a motorcar. The clamping element 2 comprises four clamps 7 projecting outwards from the housing 3, which clamps 7 are separated from each other according to two planes which are at least substantially perpendicular to each other, to enable securing the air-freshener 1 either on a vertical, or on a horizontal strip-shaped element.

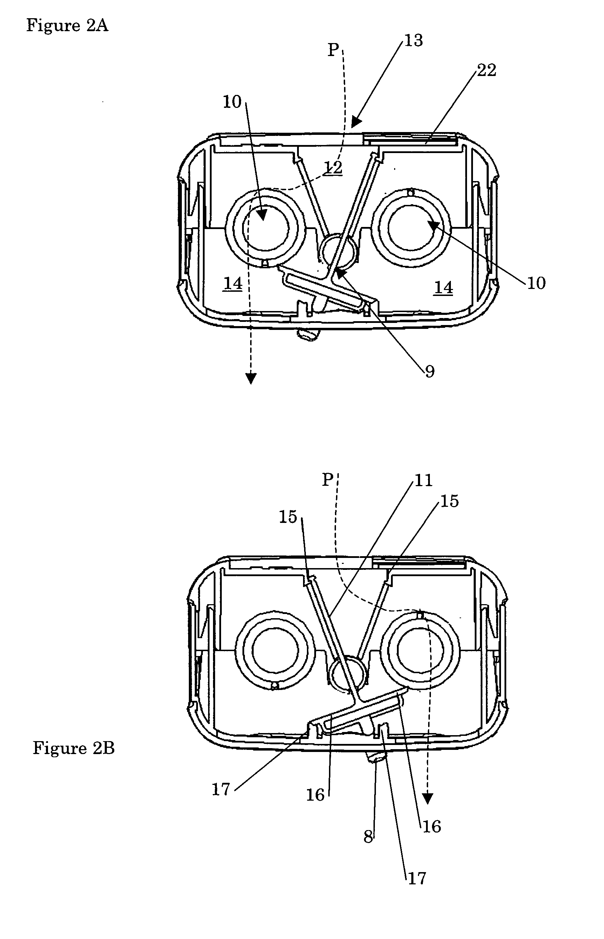

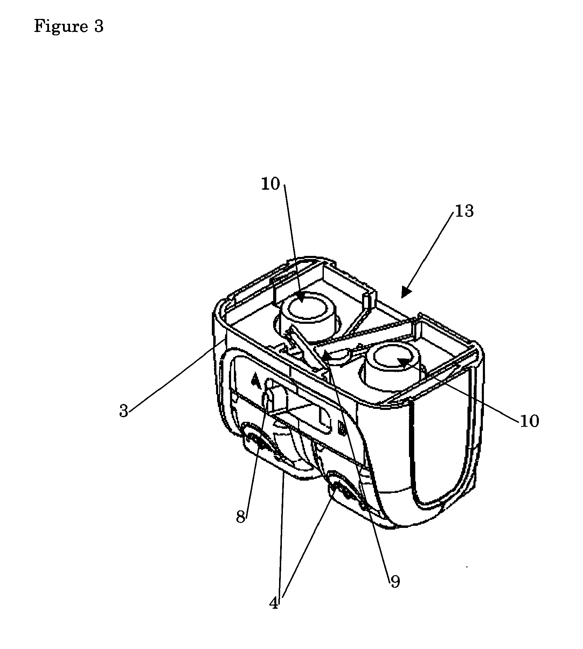

Figure 1 further shows schematically the path of the air flow indicated by arrows P. A switchable shutter element, further clarified and referenced with numeral 9 in subsequent figures, is arranged for selectively shutting access of the airflow P to any outer section of the housing 3 wherein containers 4 are provided. The shutter element is provided in a mid section between said outer sections and comprises a knob 8 protruding through the housing 3 to switch a valve piece (see Figure 2) to any of the opposite sides of the mid section as will be more clearly illustrated in Figure 2 and Figure 3.

In particular, Figures 2A and B show alternative working conditions wherein a shutter element 9 alternatively provides and shuts access to a fragrance provided via an evaporation surface in the form of a wick 10 that protrudes from containers 4 which comprise mutually different fragrances. Thus, the Figure 2A configuration provides access to the left hand wick 10, viewed from above, while shutting access to the right hand wick 10; and the Figure 2B configuration provides access to the right hand wick 10, viewed from above, while shutting access to the left hand wick 10. Furthermore, Figure 3 shows a schematic perspective cross sectional view of the dispensing device of Figure 1 sectioned along line I-I, showing the shutter in a selected shutting position.

Although in principle, via this mechanism, a mixture of fragrances can be provided by selectively opening or shutting access to any of the left and right hand wicks 10, preferably, the shutter element 9 is bistably switchable, so that only a single fragrance is activated by switching the knob 8 either to the "A" or the "B" state, to select either a fragrance from the left hand or the right hand container. In particular, Figures 2 and 3 show that preferably, the shutter element 9 comprises a valve piece 11 that is pivotably mounted in mid section 12, to include or exclude an air inlet 13 to either of outer sections 14, by pivoting the valve piece 11 against a seat structure 15 provided in the housing 3.

Furthermore, Figure 2 and Figure 3 show that the seat structure 15 is preferably wedge shaped, so that the valve piece 11 seats against either of opposite sides of the wedge shaped seat structure 15. In this wedge shape form, preferably, the valve piece comprises cross-shaped flaps 16, oriented transversely to said valve piece 11. The flaps 16, by pivoting the valve piece 11, open or close access to any of the outer sections 14 through abutting a side of a flap against a seat 17 arranged in the outer section 14.

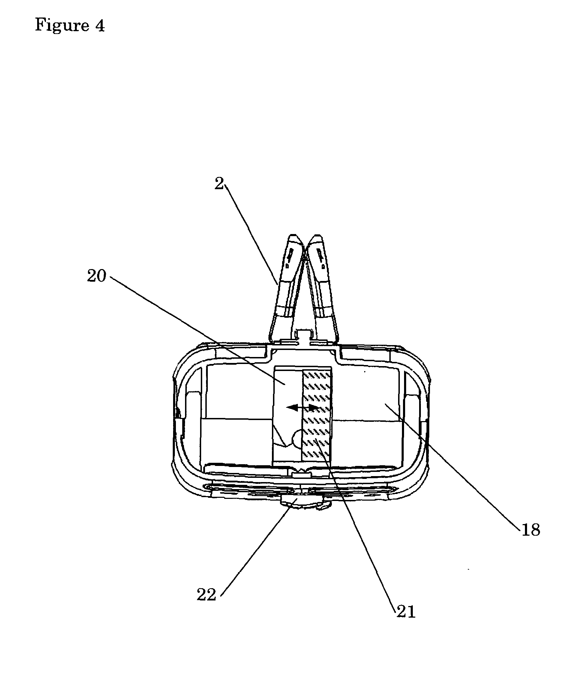

Figure 4 shows a top view of a slider 21 through a top cross sectional view of the dispenser of Figure 1 sectioned along line II-II. In particular, a lower wall 18 of a collection chamber 19 is illustrated (see also Figure 1). As can be seen in Figure 1, the collection chamber 19 is provided with an air outlet 5. Accordingly, access to this collection chamber 19 is provided via a central opening 20 that is slidably closable through slider 21. To prevent air flow near the wick 10 in a closed condition by the slider 21, additionally, preferably, the slider element 21 also slidingly closes the air inlet 13. Thus, preferably, the slider 21 comprises another closure element (not shown) slidable along an inlet opening 13 provided in a first side wall, typically, the back wall 22 (see Figure 2) of the mid section 12 forming the air inlet 13, for slidingly closing the air inlet 13 access to said mid section. This closure element is provided in addition to the closure element 21 slidable along the air outlet opening provided in a top wall 18 of the mid section 12, which is at the same time the bottom wall 18 of the collection chamber 19. Although the embodiment illustrates a slider 21 that slidingly closes the openings in a top wall (reference by 20) and a side wall of the mid section 12 (not shown), also other walls can be slidingly shutted, for example, opposite walls, according to this principle. The slider is manually operable via a slider knob 23 that is slidable along a slit 24 provided in the housing 3 (see Figure 1).

While specific embodiments of the invention have been described above, it will be appreciated that the invention may be practiced otherwise than as described. In particular, the invention is not limited to a specific design of the containers, in particular, even a single set of combined containers may be inserted in the housing, having differing sections of fragrances, without departing of the scope of the invention. In particular, the descriptions above are intended to be illustrative, not limiting. Thus, it will be apparent to one skilled in the art that modifications may be made to the invention as described without departing from the scope of the claims set out below.

## Claims

1. An air-freshening dispensing device, comprising:
- a housing, comprising a pair of mounts for mounting respective containers provided in outer sections of said housing; and
- a switchable shutter element arranged for selectively shutting access of an airflow to any of the outer sections;
- **characterized in that**
- said shutter element is provided in a mid section between said outer sections;
- said mid section being communicatively coupled to an air inlet, and further comprising an air outlet for providing throughput of the airflow through the mid section.

2. A dispensing device according to claim 1, further comprising
a clamp for clamping the unit onto an outlet channel to provide an airflow into the air inlet.

3. A dispensing unit according to claim 1, wherein the shutter element is bistably switchable.

4. A dispensing unit according to claim 1, wherein the shutter element comprises a valve piece that is mounted in the mid section, to include or exclude the air inlet to either of the outer sections, by pivoting the valve piece against a seat structure provided in the housing.

5. A dispensing unit according to claim 4, wherein the mid section is bounded by a wedge shaped seat structure, the valve piece seating against either of opposite sides of the wedge shaped seat structure; and further comprising a knob protruding through the housing to switch the valve to any of the opposite sides of the mid section.

6. A dispensing unit according to claim 5 wherein the valve piece further comprises cross-shaped flaps, oriented transversely to said valve piece.

7. A dispensing unit according to claim 6, wherein the flaps, by pivoting the valve piece, open or close access to any of the outer sections through abutting a side of a flap against a seat arranged in the outer section.

8. The dispensing unit of claim 1, further comprising a slider element that slidingly shuts the air inlet, for regulating an airflow intensity.

9. The dispensing unit of claim 8, wherein the slider comprises a closure element slidable along an inlet opening provided in a first side wall of the mid section, for slidingly closing the air inlet access to said mid section, and wherein the slider further comprises a second closure element slidable along an air outlet opening provided in a second side wall of the mid section.

10. The dispensing unit of claim 9, wherein said first and second side walls are adjacent; and wherein said second side wall is a top wall when viewed in mounted condition.

11. The dispensing unit of claim 9, wherein said first and second side walls are opposite to each other.

12. The dispensing unit of claim 10, wherein said top wall defines a lower wall of a collection chamber that is provided with an air outlet.
